Europäisches Patentamt

European Patent Office

Office européen des brevets

(11) Publication number: **0 234 760 B1**

# EUROPEAN PATENT SPECIFICATION

(45) Date of publication of patent specification: 16.09.92  (51) Int. Cl.5: **C12N 9/88**, C02F 3/12,
//(C12N9/88,C12R1:77)

(21) Application number: 87300839.5

(22) Date of filing: 30.01.87

(54) **Production of cyanide hydratase.**

(30) Priority: 19.02.86 GB 8604068

(43) Date of publication of application:
02.09.87 Bulletin 87/36

(45) Publication of the grant of the patent:
16.09.92 Bulletin 92/38

(84) Designated Contracting States:
AT BE CH DE ES FR GB GR IT LI LU NL SE

(56) References cited:
EP-A- 0 061 249
EP-A- 0 233 719

(73) Proprietor: **IMPERIAL CHEMICAL INDUSTRIES PLC**
**Imperial Chemical House, Millbank**
**London SW1P 3JF(GB)**

(72) Inventor: **Richardson, Kenneth Raymond**
**18 Ashkirk Road**
**Normanby Middlesbrough Cleveland(GB)**
Inventor: **Clarke, Peter Maurice**
**19 Prince Edward Crescent**
**Radcliffe-on-Trent Nottingham Notts(GB)**

(74) Representative: **Locke, Timothy John et al**
**ICI Group Patents Services Dept. PO Box 6**
**Shire Park Bessemer Road**
**Welwyn Garden City Herts, AL7 1HD(GB)**

EP 0 234 760 B1

**Description**

This invention relates to a process for the production of cyanide hydratase.

Certain fungi contain the enzyme cyanide hydratase EC No. 4.2.1.66 (otherwise known as formamide hydrolyase) which is capable of degrading cyanide to formamide (see for example Archives of Biochemistry and Biophysics, 151, pages 468 to 474, (1972) and Phytopathology, 67, pages 1001 to 1006, (1977)) and the following references from the Japanese J. Ferment. Technol.:-Vol. 45, No. 7, p 630-636 (1967); Vol. 46, No. 10, p 807-813 (1968); Vol. 47, No. 10, p 639-643 and 644-650 (1969) and Vol. 48, No. 5, p 277-282 and 253-290 (1970). It has been proposed to use such fungi in the microbiological treatment of cyanide containing effluents (see for example our European Patent No. 61249). To be suitable for this purpose it is desirable that the fungi contain a cyanide hydratase enzyme which is stable over a significant period of time and has a high level of activity.

According to the present invention we provide a process for the production of the enzyme cyanide hydratase which comprises the steps of (A) continuously cultivating a micro-organism strain of the genus Fusarium aerobically in an aqueous culture containing sources of carbon and of appropriate inorganic nutrients at a temperature within the range 28° to 34°C, a pH in the range 4.5 to 7.5 and a dilution rate not greater than $0.11\ hr^{-1}$ whilst continuously supplying cyanide ions and/or hydrogen cyanide under fermentation conditions to the culture at a concentration equivalent to at least 15 mM (cyanide ion and/or hydrogen cyanide in the total medium, supplied to the culture) and (B) recovering cells containing cyanide hydratase from the culture.

A method for the treatment of a cyanide-containing material to degrade the cyanide therein in which the material is treated with cyanide hydratase or a composition comprising cyanide hydratase characterised in that the cyanide hydratase has been produced by the process of the invention is also included in the scope of the invention.

The cyanide ions added to the culture become hydrogen cyanide under the conditions of the culture.

Preferably the microorganism strain is a fungal strain although suitable bacterial strains may also be used. Fungi that may be used in the process of the invention include Stemphylium loti, e.g. ATCC 11718; Mycoleptodiscus terrestris, e.g. CBS 231.53; Fusarium moniliforme, e.g. No. 3104.SA.49a available from the Canadian Department of Agriculture, Culture Collection, Ottawa, and which has also been deposited as CBS 161.82; Helminthosporium sorghicola, otherwise known as Drechslera sorghicola, e.g. CBS 249.49; Periconia circinata, e.g. CBS 263.37; and Glomerella tucamanensis, CBS 132.37. (ATCC No. refers to the number designated by the American Type Culture Collection, 12301 Park Lawn Drive, Rockville, Maryland 20852, USA, while CBS No. refers to the number designated by the Central Bureau Voor Schimmelcultures, Baarn, Netherlands). Other fungi that may be used and which have been described in the literature as producing the enzyme include Collectotrichum graminicola, Gloeocercospora sorghi, Helminthosphorium turcicum, H. maydis, H. carbonum, H. victoriae, and H. Phomna.

Preferred fungal strains are Fusarium strains, particularly Fusarium lateritium Nees strain CMI 300533, deposited 3 February 1986 at the Commonwealth Mycological Institute, Ferry Lane, Kew, Richmond, Surrey, TW9 3AF, England; and variants and mutants derived therefrom. Preferred fungal strain CMI 300533 is non-pathogenic to wheat and has the following morphological characteristics:-

Media

(1) Potato Sucrose Agar (PSA) 250 grams of potatoes washed and diced, placed in pressure cooker at 15 lbs./square inch for 15 minutes. The decoction is then squeezed through two layers of muslin, 2% of Glucose and 2% of Agar are added to the turbid filtrate and the medium autoclaved and dispersed.

(2) Czapek-Dox (Modified) Agar (Oxoid) (CDA) "Oxoid" is a Registered Trade Mark

| | |
|---|---|
| Growth conditions: | 25°C, several weeks |
| Rate of growth: | 4.0 cm. in 3 days, 3.0 cm, in 3 days respectively |
| Character of growth: | Floccose, spreading colonies with white aerial mycelium. Substratum on PSA greyish rose with patches of crimson to yellow. Tendency to be somewhat paler on CDA. Occasionally deep red pigment produced, particularly on ageing. After one to two weeks the aerial mycelium tends to become brown and collapse. The colony then becomes rather slimy as sporodochia are formed the colour being pink to brown on PSA and salmon pink on CDA. |

No exudate is formed and pigment formation tends to follow the mycelium colour.

Conidia: Microconidia not produced by this organism. Macroconidia produced from single lateral phialides or multibranched conidiophores with short phialides. In older cultures the conidiophores aggregate to form sporodochia, particularly on CDA. The conidia vary from falcate to curved fusoid dorsi-ventral, septation varying from 3 to 5, commonly 5 in youger cultures. Spore size varies from 25 -50 $\mu$ x 2.5 $\mu$ - 4.0 $\mu$.

The foot cell is often pedicellate, particularly in the longer 5 septate spores. Swollen cells occur in the mycelium and occasionally chlamydospores occur intercalary, singly or in chains.

A process for the production of the enzyme cyanide hydratase by aerobically cultivating cells of Fusarium lateritium Nees CMI 300533 is described and claimed in our co-pending UK Patent applications Nos. 8604069 and 8607595.

Fungi can be made to grow in two distinct forms, namely a ball or pellet form or in a dispersed form where the fungal cells are diffuse filamentous strands dispersed in the growth medium. For use in effluent treatment it is important that the fungus is grown in the dispersed, as opposed to ball or pellet, form. As growth in the pellet form is hampered by diffusion of nutrients and gases through the pellet, this makes cultivation inefficient. The conditions of the cultivation step (A) of the process of the invention are such as to encourage growth in the dispersed form.

In the cultivation step (A) of the process the fungal strain may suitably be grown under conditions of carbon or oxygen limitation. Preferably however the conditions are such that growth takes place effectively under dual carbon and oxygen limitation. Preferably the culture medium is a defined medium, i.e. one containing only mineral salts in addition to the carbon source without any undefined organic materials such as yeast extract.

Any suitable carbon source may be used in the process but glucose is preferred. Preferred sources of nitrogen include ammonium sulphate and ammonium hydroxide and preferred sources of phosphorus include potassium phosphate and phosphoric acid. Preferably the main constituents of the culture medium are present in the medium supplied to the process during its steady state in concentrations within the following ranges:-

| | |
|---|---|
| $H_3PO_4$ | 10 - 20 mM |
| $K_2SO_4$ | 800 - 1400 ppm |
| $MgSO_4.7H_2O$ | 700 - 1200 ppm |
| $Glucose.1H_2O$ | 10,000 - 40,000 ppm |
| $(NH_4)_2SO_4$ | 500 - 3000 ppm |
| Trace nutrients | 0.1 ppm - 20 ppm |

A very suitable culture medium to be supplied to the culture during steady state conditions has the following constitution:-

| | |
|---|---|
| 1.1M $H_3PO_4$ | 320 ml/20 L |
| Trace metals/Biotin | 10 ml/20L |
| $K_2SO_4$ | 20 g/20 L |
| $MgSO_4.7H_2O$ | 18 g/20 L |
| $Glucose.1H_2O$ | 223 g/20 L |
| $(NH_4)_2SO_4$ | 50 g/20 L |
| Trace metals/Biotin (per litre) | |

| | |
|---|---|
| $FeCl_3.6H_2O$ | 9.6 g |
| $CuSO_4.5H_2O$ | 3.6 g |
| $MnSO_4.4H_2O$ | 30.0 g |
| $ZnSO_4.7H_2O$ | 38.0 g |
| Biotin | 0.52 g |

Cyanide ions or hydrogen cyanide are supplied to the culture either separately or together with other nutrients throughout the cultivation step of the process. Preferably cyanide is added to the medium supplied to the process as alkali metal cyanides such as sodium or potassium cyanide. During start-up of the cultivation step cyanide is added initially in a low concentration which is gradually increased as the tolerance of the fungal cells in the culture towards it increases. Finally during steady state cultivation cyanide is supplied at a concentration of at least 15 mM, in the total medium supplied to the culture; i.e. the sum of all the liquid feeds to the culture, preferably 2-10 (especially 4-6) m mole/g dry cell weight. We have found that higher concentrations of cyanide ions in the medium supplied to the culture during its steady state lead to increased activity of the cyanide hydratase enzyme in the cells and that the level of enzyme activity increases linearly with increases in the cyanide ion concentration. For example enzyme activity in units of $\mu$ mole formamide produced per minute per ml of culture at a low level (5 mM) of cyanide addition is 55 whereas the enzyme activity at a higher level (20 mM) is 220 units.

Preferred conditions for the cultivation step (A) of the process are as follows:-

dilution rate in the range 0.05 to 0.1 hr$^{-1}$; pH in the range 5.0 to 6.0, especially 5.5; and temperature in the range 28$^{\circ}$ to 32$^{\circ}$C, especially 30 to 32$^{\circ}$C for highest enzyme activity.

During the cultivation step, culture is continuously removed from the fermenter in which cultivation takes place and cells containing the enzyme cyanide hydratase are separated from the removed culture by any suitable means, filtration being preferred. The separated cells may then be dried and further treated, e.g. by extrusion, to produce cyanide hydratase-containing cellular material in any suitable form, e.g. in aggregates or as a powder. Whilst the enzyme could be separated from the cells and used in cell-free form, this is generally not necessary and the enzyme is usually not separated from the cells containing it. The fungal mycelia containing the cyanide hydratase can be immobilised as described in our European Patent No. 61249 but again this refinement is generally not carried out.

The cyanide hydratase material produced by the process of the invention is very suitable for the treatment of cyanide-containing aqueous effluents, e.g. by the process of our European Patent No. 61249. The enzyme produced has a high shelf-life stability (for example half life of up to 130 days) and a high activity. Enzyme-containing material having particularly high stability is produced when the cultivation step of the process is operated under conditions of oxygen limitation or of dual oxygen and carbon limitation. Enzyme-containing material having particularly high activity is produced when the cultivation step of the process is carried out at a temperature in the range 30$^{\circ}$ to 32$^{\circ}$C.

The invention is illustrated by the following Examples:-

EXAMPLE 1

Cyanide hydratase activity was induced by growing Fusarium strain CMI 300533 in the presence of HCN in continuous culture on a glucose limiting, defined medium. Steady states were set up at different culture temperatures at a pH of 5.5 and a dilution rate of 0.10 h$^{-1}$. The results are set out in Table 1. This shows that maximal enzyme activity was induced at 30$^{\circ}$C - 32$^{\circ}$C and that growth below this temperature halved the induced activity. Growth at 34$^{\circ}$C resulted in wash-out of the culture. Enzyme activity units are $\mu$ mols of formamide produced per minute from 100 mM sodium cyanide at pH 8.5

Table 1

| Effect of temperature on activity | | | | |
|---|---|---|---|---|
| pH | Sp. act. (units/mg dry weight) | Dil. rate (h$^{-1}$) | Temp ($^{\circ}$C) | [CN] (mM) |
| 5.5 | 59.3 | 0.05 | 30.0 | 15.4 |
| 5.5 | 27.5 | 0.05 | 28.0 | 14.4 |
| 5.5 | 27.1 | 0.05 | 27.0 | 15.2 |
| 5.5 | 25.5 | 0.05 | 25.0 | 13.2 |
| 5.5 | 22.7 | 0.05 | 25.0 | 14.7 |
| 5.5 | 52.4 | 0.05 | 30.0 | 14.0 |
| 5.5 | 52.3 | 0.05 | 32.0 | 14.8 |

EXAMPLE 2.

Levels of cyanide hydratase were induced by growing Fusarium strain CMI 300533 in the presence of HCN in continuous culture on a glucose limited, defined, medium. Steady states were set up at various oxygenation rates with pH (5.5), temperature (30ºC) and dilution rate (0.10 h$^{-1}$) remaining constant. The induced enzyme was freeze dried and stored at 4ºC over silica gel and the stability of the enzyme preparations monitored by assaying aliquots of the preparations over time. The results are set out in Table 2. This shows that stability of the enzyme in storage increased when the culture was grown under dual oxygen/carbon limitation.

Table 2

| Oxygenation | 1st half-life |
|---|---|
| Aerobic | 21 days |
| mild anoxia | 63 days |
| severe anoxia | 90 days |

EXAMPLE 3.

The induction profile of cyanide hydratase was determined by growing Fusarium strain CMI 300533 in continuous culture on a glucose limited, defined, medium. Steady states were set up at various influent HCN concentrations with pH (5.5), temperature (30ºC) and dilution rate (0.10 h$^{-1}$) remaining constant.

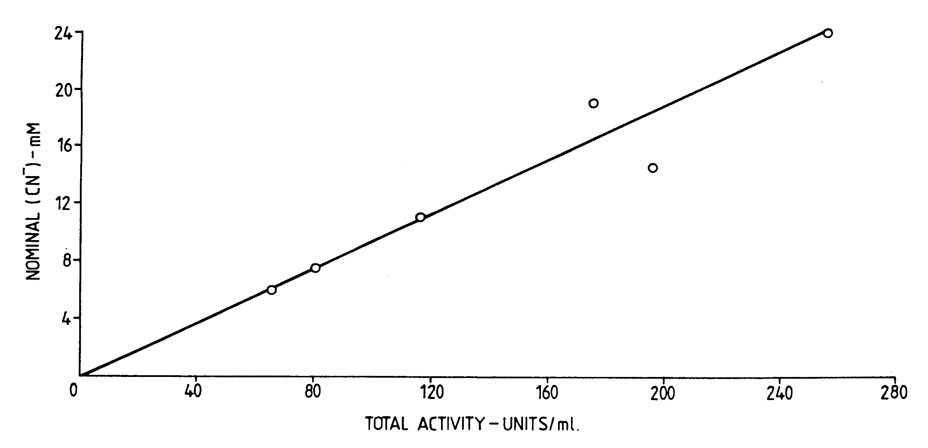

The graph shown in the drawing shows a linear response between levels of induced activity and cyanide concentration up to (at least) 24 mM cyanide in the influent medium. In the graph nominal cyanide concentration in millimolar units is plotted as ordinate with total activity (as defined above) as abscissa.

EXAMPLE 4

Fusarium strain CMI 300533 was grown continuously in a 6000 l volume vessel using a defined medium containing glucose as carbon source, the glucose concentration being growth limiting. The pH was between 5.0 and 5.8, the dilution rate was 0.08 to 0.1 hr$^{-1}$ and the temperature was 30º to 32ºC. Sodium cyanide was added to the nutrient feed at a concentration between 61.5 and 73.5 mM. The culture was subjected to oxygen stress by reduction of the aeration rate which resulted in the production of up to 0.81 g ethanol/l. Over a period of 140 hours biomass was produced at between 12.2 and 14.3 g/l with a cyanide hydratase activity of 81.0 to 122.4 $\mu$ moles formamide produced per minute per mg dry weight (assayed using 120 mM solutions of cyanide at pH 8.5 and 20ºC).

**Claims**

1. A process for the production of the enzyme cyanide hydratase which comprises the steps of (A) continuously cultivating a microorganism strain of the genus Fusarium aerobically in an aqueous culture containing sources of carbon and of appropriate inorganic nutrients at a temperature in the range 28° to 34°C, a pH in the range 4.5 to 7.5 and a dilution rate not greater than 0.11 hr$^{-1}$ whilst continuously supplying cyanide ions and/or hydrogen cyanide and/or compounds which generate cyanide ions and/or hydrogen cyanide under fermentation conditions to the culture at a concentration equivalent to at least 15 mM (cyanide ion and/or hydrogen cyanide in the total medium supplied to the culture) and (B) recovering cells containing cyanide hydratase from the culture.

2. A process according to claim 1 characterised in that the strain is Fusarium lateritium Nees strain CMI 300533 or a variant or mutant derived therefrom.

3. A process according to claim 1 or 2 characterised in that in the cultivation step (A) the microorganism strain is cultivated under conditions of carbon or oxygen limitation or of dual carbon and oxygen limitation.

4. A process according to any one of the preceding claims characterised in that the carbon source is glucose.

5. A process according to any one of the preceding claims characterised in that a source of cyanide ion and/or hydrogen cyanide is supplied to the culture at a concentration in the total medium in a range equivalent to 2-10 m mole/g dry cell weight.

6. A process according to claim 5 characterised in that the source of cyanide ion and/or hydrogen cyanide is supplied to the culture at a concentration in the total medium in a range equivalent to 4-6 m mole/g dry cell weight.

7. A process according to any one of the preceding claims characterised in that the pH is in the range 5.0 to 6.0.

8. A process according to any one of the preceding claims characterised in that the temperature is in the range 30° to 32°C.

9. A method for the treatment of a cyanide-containing material to degrade the cyanide therein in which the material is treated with cyanide hydratase or a composition comprising cyanide hydratase characterised in that the cyanide hydratase has been produced by a process as claimed in any preceding claim.

**Patentansprüche**

1. Verfahren zur Herstellung des Enzyms Cyanid-Hydratase, **dadurch gekennzeichnet**, daß es folgende Schritte umfasst:
   (A) die kontinuierliche aerobe Anzucht eines Mikroorganismusstammes des Genus Fusarium in einer wässrigen Kultur, die Kohlenstoffquellen und geeignete anorganische Nährstoffquellen bei einer Temperatur im Bereich von 28 bis 34°C, einem pH im Bereich von 4,5 bis 7,5 und einer Verdünnungsrate nicht größer als 0,11 h$^{-1}$ enthält, wobei der Kultur kontinuierlich Cyanidionen und/oder Cyanwasserstoff und/oder Verbindungen, die Cyanidionen und/oder Cyanwasserstoff erzeugen, unter Fermentationsbedingungen mit einer Konzentration, entsprechend mindestens 15 mM (an Cyanidionen und/oder Cyanwasserstoff im gesamten der Kultur zugeführten Medium) zugeführt werden und
   (B) Wiedergewinnung von Zellen aus der Kultur, die Cyanid-Hydratase enthalten.

2. Verfahren nach Anspruch 1, **dadurch gekennzeichnet**, daß der Stamm Fusarium lateritium Nees-Stamm CMI 300533 oder eine davon abgeleitete Variante oder Mutante ist.

3. Verfahren nach Anspruch 1 oder 2 **dadurch gekennzeichnet**, daß im Anzuchtsschritt (A) der Mikroorganismusstamm unter Bedingungen der Kohlenstoff- oder Sauerstofflimitation oder der zweifachen Kohlenstoff- und Sauerstofflimitation angezogen wird.

4. Verfahren nach einem der vorhergehenden Ansprüche **dadurch gekennzeichnet**, daß die Kohlenstoffquelle Glucose ist.

5. Verfahren nach einem der vorhergehenden Ansprüche **dadurch gekennzeichnet**, daß eine Cyanidionenquelle und/oder eine Cyanwasserstoffquelle der Kultur in einer Konzentration im Gesamtmedium im Bereich entsprechend 2 bis 10 mmol/g Zelltrockenmasse zugeführt wird.

6. Verfahren nach Anspruch 5 **dadurch gekennzeichnet**, daß die Cyanidionenquelle und/oder die Cyanwasserstoffquelle der Kultur in einer Konzentration im Gesamtmedium im Bereich entsprechend 4 bis 6 mmol/g Zelltrockenmasse zugeführt wird.

7. Verfahren nach einem der vorhergehenden Ansprüche **dadurch gekennzeichnet**, daß der pH im Bereich von 5,0 bis 6,0 liegt.

8. Verfahren nach einem der vorhergehenden Ansprüche **dadurch gekennzeichnet**, daß die Temperatur im Bereich von 30 bis 32°C liegt.

9. Verfahren zur Behandlung eines cyanidionenenthaltenden Stoffes zur Zersetzung des Cyanids, wobei der Stoff mit Cyanid-Hydratase oder einer Zusammensetzung, die Cyanid-Hydratase umfasst, behandelt wird **dadurch gekennzeichnet**, daß die Cyanid-Hydratase mit einem Verfahren nach einem der vorhergehenden Ansprüche hergestellt wurde.

**Revendications**

1.  Procédé pour la production de la cyanure hydratase qui comprend les étapes de (A) culture en continu d'une souche de microorganisme du genre Fusarium de façon aérobie dans une culture aqueuse contenant des sources de carbone et d'éléments nutritifs inorganiques appropriés à une température dans la gamme de 28° à 34°C, à un pH de l'ordre de 4,5 à 7,5 et avec une vitesse de dilution ne dépassant pas 0,11 h$^{-1}$, avec introduction en continu dans la culture d'ions cyanure et/ou d'acide cyanhydrique et/ou de composés qui génèrent des ions cyanure et/ou de l'acide cyanhydrique dans des conditions de fermentation à une concentration équivalente à au moins 15 mM (ion cyanure et/ou acide cyanhydrique dans le milieu total fourni à la culture) et (B) récupération des cellules contenant la cyanure hydratase à partir de la culture.

2.  Procédé suivant la revendication 1, caractérisé en ce que la souche est la souche Fusarium lateritium Nees CMI 300533 ou un variant ou mutant dérivé de celle-ci.

3.  Procédé suivant la revendication 1 ou 2, caractérisé en ce que dans l'étape de culture (A), la souche de microorganisme est cultivée dans des conditions de limitation en carbone ou en oxygène ou de limitation double en carbone et oxygène.

4.  Procédé suivant l'une quelconque des revendications précédentes, caractérisé en ce que la source de carbone est du glucose.

5.  Procédé suivant l'une quelconque des revendications précédentes, caractérisé en ce qu'une source d'ion cyanure et/ou d'acide cyanhydrique est fournie à la culture à une concentration dans le milieu total dans une gamme équivalente à 2 - 10 mmoles/g de poids de cellule sèche.

6.  Procédé suivant la revendication 5, caractérisé en ce que la source d'ion cyanure et/ou d'acide cyanhydrique est fournie à la culture à une concentration dans le milieu total dans une gamme équivalente à 4 - 6 mmoles/g de poids de cellule sèche.

7.  Procédé suivant l'une quelconque des revendications précédentes, caractérisé en ce que le pH est de l'ordre de 5,0 à 6,0.

8.  Procédé suivant l'une quelconque des revendications précédentes, caractérisé en ce que la température est de l'ordre de 30° à 32°C.

9.  Procédé pour le traitement d'une matière contenant du cyanure pour dégrader le cyanure qu'elle contient selon lequel la matière est traitée avec une cyanure hydratase ou une composition comprenant une cyanure hydratase , caractérisé en ce que la cyanure hydratase a été produite par un procédé suivant l'une quelconque des revendications précédentes.